# EUROPEAN PATENT APPLICATION

(11) **EP 4 817 409 A1**
(43) Date of publication of application: **07.10.2026**
(21) Application number: 24897674.8
(22) Date of filing: 29.11.2024
(51) Int. Cl.: A01H 6/14, A01H 1/06, A23L 33/105, C07H 15/256, C12N 5/04, C12P 19/56, C12Q 1/6895

(54) **STEVIA PLANT HAVING HIGH REBAUDIOSIDE M CONTENT**

(30) Priority: 01.12.2023 JP 2023204266
(71) Applicant: Suntory Holdings Limited, Osaka-shi, Osaka 530-8203 (JP)
(72) Inventor: HIRAI Tadayoshi, Soraku-gun, Kyoto 619-0284 (JP); IWAKI Kazunari, Kawasaki-shi, Kanagawa 211-0067 (JP); OCHIAI Kentaro, Soraku-gun, Kyoto 619-0284 (JP); MIYAGAWA Katsuro, Soraku-gun, Kyoto 619-0284 (JP); OKADA Emi, Soraku-gun, Kyoto 619-0284 (JP); KOMAKI Maki, Soraku-gun, Kyoto 619-0284 (JP)
(74) Representative: V.O.
(86) International application number: PCT/JP2024/042255
(87) International publication number: WO 2025/115990

(57) **Abstract**

Provided is a stevia plant having at least one of genetic characteristics (1) to (5).

## Description

### Technical Field

The present invention relates to a stevia plant having a high content of a useful steviol glycoside such as rebaudioside M, a method of screening for the same, and the like.

### Background Art

A variety of beverages have been developed and marketed to meet the increasing needs of consumers. Sugars such as sucrose are components which are usually blended in beverages for the purpose of imparting sweetness and the like. However, the influence on health due to excessive intake has been pointed out, and there is an increasing need for sweeteners which have lower calories and are derived from natural sources. For example, Patent Literature 1 discloses a functional sweetener composition containing a vitamin, a high-intensity sweetener, and a sweetness improving composition.

A steviol glycoside is known as a sweet component contained in a stevia extract. The stevia extract is mainly extracted and purified from leaves of stevia. Stevia is an asteraceous perennial plant originated from Paraguay in South America and having a scientific name of Stevia Rebaudiana Bertoni. Stevia contains a component having a sweetness of about 300 times or more that of sugar, and thus is cultivated to extract this sweet component and use it as a natural sweetener. As the steviol glycoside, the existence of various glycosides such as rebaudioside A (hereinafter, the "rebaudioside" is sometimes abbreviated as "Reb"), RebB, RebC, RebD, RebE, RebM, and RebN has been reported (Patent Literature 2).

Under such circumstances, a stevia plant containing 1.53% of RebM per dried leaf is known (Patent Literature 3).

### Citation List

### Patent Literature

Patent Literature 1: WO2007/070224
Patent Literature 2: WO2010/038911
Patent Literature 3: WO2021/230256

### Disclosure of the Invention

### Technical Problem

Since RebM cannot be obtained in large amounts from natural stevia plants, efficient availability thereof is required.

### Solution to Problem

The present invention provides a stevia plant having a higher content of a useful steviol glycoside such as RebM than that in a wild-type stevia species, a method of producing the plant, and a method of screening for the plant.

In one aspect, the present invention provides the following.
[1] A stevia plant having at least one of the following genetic features (1) to (5).
   (1) Being homozygous or heterozygous for the allele in which the sequence of a portion corresponding to positions 150 to 151 of SEQ ID NO: 1 is T.
   (2) Being homozygous or heterozygous for the allele in which the base at a position corresponding to position 151 of SEQ ID NO: 2 is TAGGTGGTGACACTGTAGC.
   (3) Being homozygous or heterozygous for the allele in which the base at a position corresponding to position 151 of SEQ ID NO: 3 is A.
   (4) Being homozygous or heterozygous for the allele in which the base at a position corresponding to position 151 of SEQ ID NO: 4 is A.
   (5) Being homozygous for the allele in which the base at a position corresponding to position 151 of SEQ ID NO: 5 is A.
[2] A stevia plant having at least one of the following chemical features (i) to (v).
   (i) A content of rebaudioside (Reb) M is 1.8% or more per unit mass of a dried leaf.
   (ii) A difference between the content of RebM and a content of stevioside (ST) is 0.9% or more per unit mass of a dried leaf.
   (iii) A difference between the content of RebM and a content of RebE is 1.3% or more per unit mass of a dried leaf.
   (iv) a ratio RebM/RebN of the content of RebM to a content of RebN is 1.52 or more per unit mass of a dried leaf.
   (v) a ratio RebM/stevioside of the content of RebM to the content of stevioside is 1.83 or more per unit mass of a dried leaf.
[3] The plant according to [1] or [2], wherein the plant comprises a stevia plant subjected to a mutagenesis treatment and a progeny plant thereof.
[4] A dead tissue or dead cell of the plant according to any of [1] to [3].
[5] A seed, tissue, dried leaf, tissue culture, or cell of the plant according to any of [1] to [3].
[6] The tissue, tissue culture, or cell according to [5], which is selected from embryos, meristematic cells, pollen, leaves, roots, root tips, petals, protoplasts, leaf sections and callus.
[7] A method of producing the stevia plant according to any of [1] to [3], the method comprising a step of crossing the plant according to any of [1] to [3] with a second stevia plant.
[8] A method of producing the stevia plant according to any of [1] to [3], the method comprising a step of introducing, into a genome of a stevia plant, at least one of the following mutations.
   (1) A mutation of the sequence of a portion corresponding to positions 150 to 151 of SEQ ID NO: 1 to T.
   (2) A mutation of the base at a position corresponding to position 151 of SEQ ID NO: 2 to TAGGTGGTGACACTGTAGC.
   (3) A mutation of the base at a position corresponding to position 151 of SEQ ID NO: 3 to A.
   (4) A mutation of the base at a position corresponding to position 151 of SEQ ID NO: 4 to A.
   (5) A mutation of the base at a position corresponding to position 151 of SEQ ID NO: 5 to A.
[9] An extract of the plant according to any one of [1] to [3], of the dead tissue or dead cell according to [4], of the seed, tissue, dried leaf, tissue culture, or cell according to [5], or of the tissue, tissue culture, or cell according to [6].
[10] A method of producing a steviol glycoside-containing extract, the method comprising a step of obtaining an extract from the plant according to any one of [1] to [3], from the dead tissue or dead cell according to [4], from the seed, tissue, dried leaf, tissue culture, or cell according to [5], or from the tissue, tissue culture, or cell according to [6].
[11] A method of producing a steviol glycoside, the method comprising a step of purifying the steviol glycoside from the extract according to [9].
[12] A method of producing a food or beverage, a sweetener composition, a flavor, or a medicament, the method comprising:
   a step of providing an extract of the plant according to any one of [1] to [3], an extract of the dead tissue or dead cell according to [4], an extract of the seed, tissue, dried leaf, tissue culture, or cell according to [5], an extract of the tissue, tissue culture, or cell according to [6], or the extract according to [9]; and
   a step of adding the extract to a raw material for the food or beverage, the sweetener composition, the flavor, or the medicament.
[13] A food or beverage, a sweetener composition, a flavor, or a medicament, containing an extract of the plant according to any one of [1] to [3], an extract of the dead tissue or dead cell according to [4], an extract of the seed, tissue, dried leaf, tissue culture, or cell according to [5], an extract of the tissue, tissue culture, or cell according to [6], or the extract according to [9].
[14] A method of producing a food or beverage, a sweetener composition, a flavor, or a medicament, the method comprising:
   a step of purifying a steviol glycoside from the extract according to [9]; and
   a step of adding the purified steviol glycoside to a raw material for the food or beverage, the sweetener composition, the flavor, or the medicament.
[15] A method of screening for a stevia plant having at least one of the chemical features (i) to (v) recited in [2], the method comprising a step of detecting, in a test stevia plant, the presence and/or absence of at least one of the genetic features (1) to (5) recited in [1].
[16] A screening kit for a stevia plant having at least one of the chemical features (i) to (v) recited in [2], the kit comprising a reagent for detecting the presence and/or absence of at least one of the genetic features (1) to (5) recited in [1].

### Advantageous Effects of Invention

The present invention enables the obtainment of a stevia plant having a useful steviol glycoside in a larger amount and the provision of a means for producing such a plant, a leaf obtained from such a plant, and a food, a beverage, etc., containing a useful steviol glycoside obtained from this leaf.

### Brief Description of Drawings

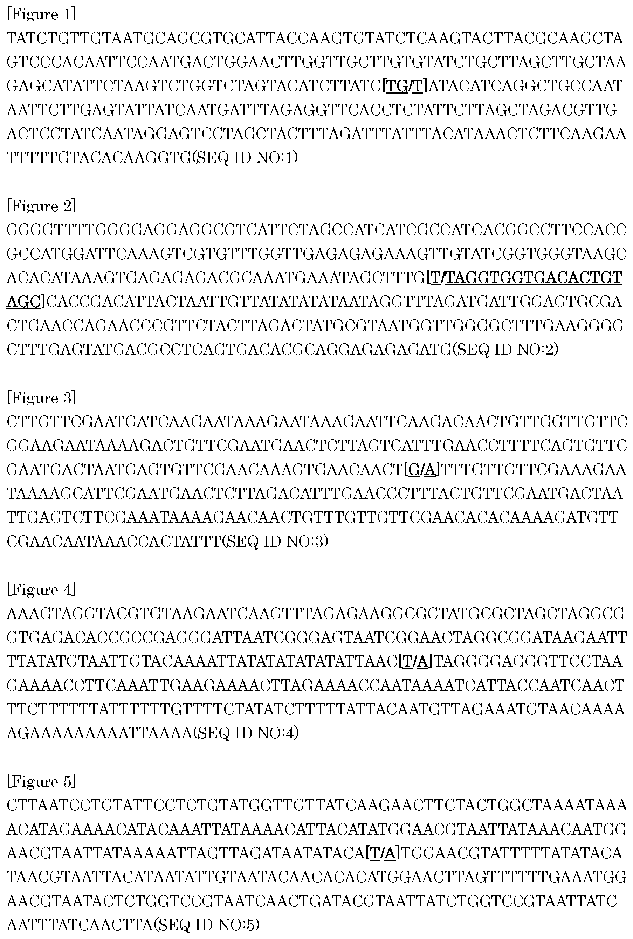

[FIG. 1] FIG. 1 is a diagram showing a position of a mutation according to a genetic feature (1) in a nucleotide sequence of SEQ ID NO: 1. The bases on the left side of a slash in parentheses represent a wild-type sequence and those on the right side represent a variant sequence.
[FIG. 2] FIG. 2 is a diagram showing a position of a mutation according to a genetic feature (2) in a nucleotide sequence of SEQ ID NO: 2. The base on the left side of a slash in parentheses represent a wild-type sequence and those on the right side represent a variant sequence.
[FIG. 3] FIG. 3 is a diagram showing a position of a mutation according to a genetic feature (3) in a nucleotide sequence of SEQ ID NO: 3. The base on the left side of a slash in parentheses represent a wild-type sequence and those on the right side represent a variant sequence.
[FIG. 4] FIG. 4 is a diagram showing a position of a mutation according to a genetic feature (4) in a nucleotide sequence of SEQ ID NO: 4. The base on the left side of a slash in parentheses represent a wild-type sequence and those on the right side represent a variant sequence.
[FIG. 5] FIG. 5 is a diagram showing a position of a mutation according to a genetic feature (5) in a nucleotide sequence of SEQ ID NO: 5. The base on the left side of a slash in parentheses represent a wild-type sequence and those on the right side represent a variant sequence.
[FIG. 6] FIG. 6 illustrates results of analyzing whether or not a stevia plant has the genetic features (2'), (4'), or (5).

### Description of Embodiments

The present invention will now be described in detail. The following embodiments are provided for illustrating the present invention and are not intended to limit the present invention only thereto. The present invention may be implemented in various forms, without departing from the spirit of the present invention.

Note that all documents, as well as laid-open application publications, patent application publications, and other Patent Literatures cited herein shall be incorporated herein by reference. The present specification incorporates the contents of the specification and the drawings of Japanese Patent Application (No. 2023-204266), filed on December 1, 2023, from which the present application claims priority.

### 1. Plant of present invention

### 1-1. Stevia plant having at least one of genetic features (1) to (5)

In one aspect, the present invention provides a stevia plant having at least one of the following genetic features (1) to (5) (hereinafter sometimes referred to as "plant A of the present invention" or "stevia plant A of the present invention").
(1) Being homozygous or heterozygous for the allele in which the sequence of a portion corresponding to positions 150 to 151 of SEQ ID NO: 1 is T.
(2) Being homozygous or heterozygous for the allele in which the base at a position corresponding to position 151 of SEQ ID NO: 2 is TAGGTGGTGACACTGTAGC.
(3) Being homozygous or heterozygous for the allele in which the base at a position corresponding to position 151 of SEQ ID NO: 3 is A.
(4) Being homozygous or heterozygous for the allele in which the base at a position corresponding to position 151 of SEQ ID NO: 4 is A.
(5) Being homozygous for the allele in which the base at a position corresponding to position 151 of SEQ ID NO: 5 is A.

In a preferred aspect, the genetic features (1) to (4) are heterozygous as follows.
(1') Being heterozygous for the allele in which the sequence of a portion corresponding to positions 150 to 151 of SEQ ID NO: 1 is T.
(2') Being heterozygous for the allele in which the base at a position corresponding to position 151 of SEQ ID NO: 2 is TAGGTGGTGACACTGTAGC.
(3') Being heterozygous for the allele in which the base at a position corresponding to position 151 of SEQ ID NO: 3 is A.
(4') Being heterozygous for the allele in which the base at a position corresponding to position 151 of SEQ ID NO: 4 is A.

The phrase "position/portion corresponding to" means the following. In case a sequence identical to a reference sequence (e.g., SEQ ID NOs: 1 to 5) is present in a genome, it means a position/portion (for example, positions 150 to 151, 151, or the like) in the sequence present in the genome. In case a sequence identical to the reference sequence is not present in a genome, it means a position/portion in a sequence corresponding to the reference sequence in the genome, which corresponds to the position/portion in the reference sequence. Whether or not a sequence identical to or corresponding to the reference sequence is present in the genome can be determined by, for example, amplifying genomic DNA of a stevia plant of interest with a primer capable of amplifying the reference sequence by PCR, sequencing the amplified product, and performing alignment analysis between the obtained sequence and the reference sequence. Non-limiting examples of the sequence corresponding to the reference sequence include, for example, a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more, or 99.8% or more to the reference sequence. The position/portion in the sequence corresponding to the reference sequence in the genome, which corresponds to the position/portion in the reference sequence, can be determined by taking into account the nucleotide sequences before and after the position/portion in the reference sequence, and the like. For example, the position/portion in the sequence corresponding to the reference sequence in the genome, which corresponds to the position/portion in the reference sequence, can be determined by alignment analysis of the reference sequence with the sequence corresponding to the reference sequence in the genome.

For instance, the "portion corresponding to positions 150 to 151 of SEQ ID NO: 1" of the genetic feature (1) of the present invention is taken as an example. When the genome of a stevia plant has a portion consisting of the nucleotide sequence identical to SEQ ID NO: 1, the "portion corresponding to positions 150 to 151 of SEQ ID NO: 1" is positions 150 to 151 from the 5' end side of the portion consisting of the nucleotide sequence identical to SEQ ID NO: 1 in the genome. On the other hand, when the genome of a stevia plant has a portion consisting of a nucleotide sequence that is not identical to but corresponds to SEQ ID NO: 1, the genome does not have a portion consisting of a nucleotide sequence identical to SEQ ID NO: 1, and thus the "portion corresponding to positions 150 to 151 of SEQ ID NO: 1" does not necessarily correspond to positions 150 to 151 from the 5' end side of the portion corresponding to SEQ ID NO: 1. However, the "portion corresponding to positions 150 to 151 of SEQ ID NO: 1" in the genome of such a stevia plant can be identified by taking into account the nucleotide sequences before and after the positions 150 to 151 of SEQ ID NO: 1, and the like. For example, the "portion corresponding to positions 150 to 151 of SEQ ID NO: 1 " in the genome of the stevia plant can be identified by alignment analysis of the nucleotide sequence of the portion corresponding to SEQ ID NO: 1 in the genome of the stevia plant with the nucleotide sequence of SEQ ID NO: 1.

Here, positions selected from the group consisting of (1) the portion corresponding to positions 150 to 151 of SEQ ID NO: 1, (2) the position corresponding to position 151 of SEQ ID NO: 2, (3) the position corresponding to position 151 of SEQ ID NO: 3, (4) the position corresponding to position 151 of SEQ ID NO: 4, and (5) the position corresponding to position 151 of SEQ ID NO: 5 are sometimes collectively referred to as "polymorphic site of the present invention" or "mutation site of the present invention". In addition, each of the positions/portions (1) to (5) described above is sometimes referred to as "polymorphic site (1)", "polymorphic site (2)", "mutation site (1)", "mutation site (2)", or the like.

"The sequence of a portion corresponding to SEQ ID NO: 1" means, for instance, a portion consisting of a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more, or 99.8% or more to the nucleotide sequence of SEQ ID NO: 1.

In some aspects, the "portion consisting of a nucleotide sequence corresponding to SEQ ID NO: 1" includes a portion of the genome of the stevia plant which can be amplified by PCR using a forward primer that hybridizes to a complementary sequence of a portion of positions 1 to 149 from the 5' end of SEQ ID NO: 1 (i.e., from the 5' end of SEQ ID NO: 1 to bases on the single-base 5' end side of the mutation site (1)) and a reverse primer that hybridizes to a portion of positions 1 to 150 from the 3' end side of SEQ ID NO: 1 (i.e., from the 3' end of SEQ ID NO: 1 to bases on the single-base 3' end side of the mutation site (1)).

Here, for simplicity, the genetic feature (1) of the present invention is described as an example, but the same applies to the genetic features (2) to (5) of the present invention.

In a specific aspect, the "portion consisting of a nucleotide sequence corresponding to SEQ ID NO: 1" includes, for example, a portion of the genome of the stevia plant which can be amplified by PCR using the forward primer containing the nucleotide sequence of SEQ ID NO: 6 and the reverse primer containing the nucleotide sequence of SEQ ID NO: 7.

In a specific aspect, the "portion consisting of a nucleotide sequence corresponding to SEQ ID NO: 2" includes, for example, a portion of the genome of the stevia plant which can be amplified by PCR using the forward primer containing the nucleotide sequence of SEQ ID NO: 8 and the reverse primer containing the nucleotide sequence of SEQ ID NO: 9.

In a specific aspect, the "portion consisting of a nucleotide sequence corresponding to SEQ ID NO: 4" includes, for example, a portion of the genome of the stevia plant which can be amplified by PCR using the forward primer containing the nucleotide sequence of SEQ ID NO: 10 and the reverse primer containing the nucleotide sequence of SEQ ID NO: 11.

In a specific aspect, the "portion consisting of a nucleotide sequence corresponding to SEQ ID NO: 5" includes, for example, a portion of the genome of the stevia plant which can be amplified by PCR using the forward primer containing the nucleotide sequence of SEQ ID NO: 12 and the reverse primer containing the nucleotide sequence of SEQ ID NO: 13.

In a specific aspect, the "allele in which the sequence of a portion corresponding to positions 150 to 151 of SEQ ID NO: 1 is T" contains a nucleotide sequence of SEQ ID NO: 14, 15, 16, or 17.

In a specific aspect, the "allele in which the base at a position corresponding to position 151 of SEQ ID NO: 2 is TAGGTGGTGACACTGTAGC" contains a nucleotide sequence of SEQ ID NO: 18, 19, 20, or 21.

In a specific aspect, the "allele in which the base at a position corresponding to position 151 of SEQ ID NO: 3 is A" contains a nucleotide sequence of SEQ ID NO: 22, 23, 24, or 25.

In a specific aspect, the "allele in which the base at a position corresponding to position 151 of SEQ ID NO: 4 is A" contains a nucleotide sequence of SEQ ID NO: 26, 27, 28, or 29.

In a specific aspect, the "allele in which the base at a position corresponding to position 151 of SEQ ID NO: 5 is A" contains a nucleotide sequence of SEQ ID NO: 30, 31, 32, or 33.

In addition, mutations selected from the group consisting of (1) a mutation from TG to T in a portion corresponding to positions 150 to 151 of SEQ ID NO: 1, (2) a mutation from T to TAGGTGGTGACACTGTAGC at a position corresponding to position 151 of SEQ ID NO: 2, (3) a mutation from G to A at a position corresponding to position 151 of SEQ ID NO: 3, (4) a mutation from T to A at a position corresponding to position 151 of SEQ ID NO: 4, and (5) a mutation from T to A at a position corresponding to position 151 of SEQ ID NO: 5 are sometimes collectively referred to as "polymorphism of the present invention" or "mutation of the present invention". In addition, each of the mutations (1) to (5) described above is sometimes referred to as "polymorphism (1) of the present invention", "polymorphism (2) of the present invention", "mutation (1) of the present invention", "mutation (2) of the present invention", or the like.

The genetic features described above can be detected by PCR method, TaqMan PCR method, sequencing method, microarray method, Invader method, TILLING method, RAD (random amplified polymorphic DNA) method, restriction fragment length polymorphism (RFLP) method, PCR-SSCP method, AFLP (amplified fragment length polymorphism) method, SSLP (simple sequence length polymorphism) method, CAPS (cleaved amplified polymorphic sequence) method, dCAPS (derived cleaved amplified polymorphic sequence) method, allele-specific oligonucleotide (ASO) method, ARMS method, denaturing gradient gel electrophoresis (DGGE) method, CCM (chemical cleavage of mismatch) method, DOL method, MALDI-TOF/MS method, TDI method, padlock probe method, molecular beacon method, DASH (dynamic allele specific hybridization) method, UCAN method, ECA method, PINPOINT method, PROBE (primer oligo base extension) method, VSET (very short extension) method, Survivor assay, Sniper assay, Luminex assay, GOOD method, LCx method, SNaPshot method, Mass ARRAY method, pyrosequencing method, SNP-IT method, melting curve analysis method, etc., but detection methods are not limited thereto.

In a specific aspect, the genetic features of the present invention can be detected by the dCAPS method using a combination of the following primer sets and restriction enzymes.

When candidate plants have a mutation (1'), for example, only a band of about 144 bp length (for example, SEQ ID NO: 36) can be obtained even when genomic DNAs of the candidate plants are subjected to PCR amplification using a forward primer having a nucleotide sequence shown in SEQ ID NO: 34 and a reverse primer having a nucleotide sequence shown in SEQ ID NO: 35 and the resulting PCR products (about 144 bp length, for example, SEQ ID NO: 36) are treated with the restriction enzyme Hpy188I. On the other hand, when candidate plants do not have the mutation (1'), a band of about 117 bp length (for example, SEQ ID NO: 38) and a band of about 28 bp length (for example, SEQ ID NO: 39) can be obtained when PCR amplification is performed in the same manner as described above and the resulting PCR products (about 145 bp length, for example, SEQ ID NO: 37) are treated with the restriction enzyme Hpy188I. Therefore, when a band of about 144 bp length is observed by the dCAPS method, it can be determined that the candidate plants have the genetic features (1') of the present invention.

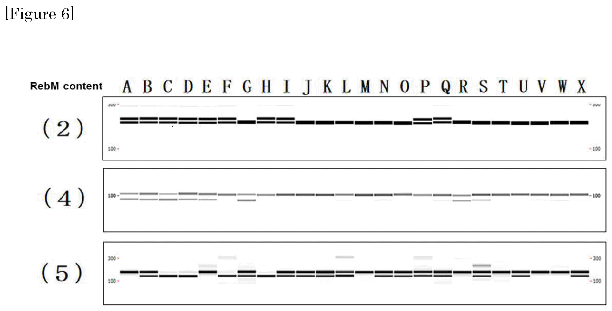

When candidate plants have a mutation (4'), for example, a band of about 78 bp length (for example, SEQ ID NO: 43) and a band of about 25 bp length (for example, SEQ ID NO: 44) can be obtained when genomic DNAs of the candidate plants are subjected to PCR amplification using a forward primer having a nucleotide sequence shown in SEQ ID NO: 40 and a reverse primer having a nucleotide sequence shown in SEQ ID NO: 41 and the resulting PCR products (about 103 bp length, for example, SEQ ID NO: 42) are treated with the restriction enzyme NdeI. On the other hand, when candidate plants do not have the mutation (4'), only a band of about 103 bp length (for example, SEQ ID NO: 45) can be obtained even when PCR amplification is performed in the same manner as described above and the resulting PCR products (about 103 bp length, for example, SEQ ID NO: 45) are treated with the restriction enzyme NdeI. Therefore, when a band of about 78 bp length and/or a band of about 25 bp length derived from a degraded product and a band of about 103 bp length derived from a non-degraded product are observed by the dCAPS method, it can be determined that the candidate plants have the genetic features (4') of the present invention. FIG. 6 illustrates an example in which the genetic feature (4') was detected by the approach described above.

When candidate plants have a mutation (5'), for example, a band of about 130 bp length (for example, SEQ ID NO: 49) and a band of about 31 bp length (for example, SEQ ID NO: 50) can be obtained when genomic DNAs of the candidate plants are subjected to PCR amplification using a forward primer having a nucleotide sequence shown in SEQ ID NO: 46 and a reverse primer having a nucleotide sequence shown in SEQ ID NO: 47 and the resulting PCR products (about 161 bp length, for example, SEQ ID NO: 48) are treated with the restriction enzyme Mfel. On the other hand, when candidate plants do not have the mutation (5'), only a band of about 161 bp length (for example, SEQ ID NO: 51) can be obtained even when PCR amplification is performed in the same manner as described above and the resulting PCR products (about 161 bp length, for example, SEQ ID NO: 51) are treated with the restriction enzyme NdeI. Therefore, when a band of about 130 bp length and/or a band of about 31 bp length derived from a degraded product and a band of about 161 bp length derived from a non-degraded product are observed by the dCAPS method, it can be determined that the candidate plants have the genetic features (5') of the present invention. FIG. 6 illustrates an example in which the genetic feature (5') was detected by the approach described above.

When candidate plants have a mutation (2'), for example, PCR products (about 206 bp length, for example, SEQ ID NO: 54) can be obtained when genomic DNAs of the candidate plants are subjected to PCR amplification using a forward primer having a nucleotide sequence shown in SEQ ID NO: 52 and a reverse primer having a nucleotide sequence shown in SEQ ID NO: 53. On the other hand, when candidate plants do not have the mutation (2'), PCR products (about 188 bp length, for example, SEQ ID NO: 55) can be obtained when PCR amplification is performed in the same manner as described above. Therefore, when a band of about 206 bp length and a band of about 188 bp length are observed, it can be determined that the candidate plants have the genetic features (2') of the present invention. FIG. 6 illustrates an example in which the genetic feature (2') was detected by the approach described above.

In some aspects, the plant A of the present invention has at least one property selected from an RebM content, a ratio of the RebM content to an RebN content (RebM/RebN), a ratio of the RebM content to an ST content (RebM/ST), RebM - ST (difference between the RebM content and the ST content, the same applies hereinafter), and RebM - RebE, which is high as compared to that of a stevia plant (hereinafter sometimes referred to as a control plant) which does not have any of the genetic features (1'), (2'), (4') and (5).

The phrase "higher than that in a control plant" means that, for example, when the plants are cultivated under the same cultivation conditions, the content of the above component (e.g., the content in a dried leaf) or the content ratio or content difference is higher, preferably statistically significantly higher, than that in the control plant. More specifically, it means that, for example, when the plants are cultivated under the same cultivation conditions, the content of the above component, the content ratio or the content difference in a dried leaf is more than 1-fold, about 1.1-fold or more, about 1.2-fold or more, about 1.3-fold or more, about 1.4-fold or more, about 1.5-fold or more, about 1.6-fold or more, about 1.7-fold or more, about 1.8-fold or more, about 1.9-fold or more, about 2-fold or more, about 2.1-fold or more, about 2.2-fold or more, about 2.3-fold or more, about 2.4-fold or more, about 2.5-fold or more, about 2.6-fold or more, about 2.7-fold or more, about 2.8-fold or more, about 2.9-fold or more, about 3-fold or more, about 3.1-fold or more, about 3.2-fold or more, about 3.3-fold or more, about 3.4-fold or more, about 3.5-fold or more, about 3.6-fold or more, about 3.7-fold or more, about 3.8-fold or more, about 3.9-fold or more, about 4-fold or more, about 4.1-fold or more, about 4.2-fold or more, about 4.3-fold or more, about 4.4-fold or more, about 4.5-fold or more, about 4.6-fold or more, about 4.7-fold or more, about 4.8-fold or more, about 4.9-fold or more, about 5-fold or more, about 6-fold or more, about 7-fold or more, about 8-fold or more, about 9-fold or more, about 10-fold or more, about 15-fold or more, about 20-fold or more, about 25-fold or more, about 30-fold or more, about 35-fold or more, about 40-fold or more, about 45-fold or more, about 50-fold or more, about 55-fold or more, about 60-fold or more, about 65-fold or more, about 70-fold or more, about 75-fold or more, about 80-fold or more, about 85-fold or more, about 90-fold or more, about 95-fold or more, or about 100-fold or more of the content, content ratio or content difference of the control plant. In addition, for example, it means that when the content of the above component, the content ratio or the content difference in a dried leaf in a population of the plant A of the present invention and the content of the above component, the content ratio or the content difference in a dried leaf in a population of the control plant are tested by the Welch's t-test, an average value of the former is larger than an average value of the latter, and a P value in a two sided test is less than 0.05, preferably less than 0.01, and particularly preferably less than 0.005. The contents and the like to be compared may be average values for a plurality of individuals.

The total steviol glycoside (TSG) is a generic term for measurable steviol glycosides and does not include unknown steviol glycosides or steviol glycosides present in amounts less than the limit of detection. Preferably, the TSG is any combination of two or more selected from the group consisting of RebA, RebB, RebC, RebD, RebE, RebF, RebG, RebI, RebJ, RebK, RebM, RebN, RebO, RebQ, RebR, dulcoside A, rubusoside, steviolmonoside, steviolbioside, and stevioside. In a specific aspect, the TSG consists of RebA, RebB, RebC, RebD, RebE, RebF, RebI, RebJ, RebM, RebN, and stevioside.

In some aspects, the plant A of the present invention may have a plurality of the genetic features (1) to (5) described above. In the aspects, the number of genetic features may be any of from 2 to 5, i.e., 2, 3, 4, or 5. In a specific aspect, the plant A of the present invention has the genetic features (1'), (2'), (4'), and (5).

### 1-2. Stevia plant having at least one of chemical features (i) to (v)

In one aspect, the present invention provides a stevia plant having at least one of the following chemical features (i) to (v) (hereinafter sometimes collectively referred to as "plant B of the present invention" or "stevia plant B of the present invention").
(i) A content of RebM is 1.8% or more per unit mass of a dried leaf.
(ii) A difference between the content of RebM and a content of stevioside is 0.9% or more per unit mass of a dried leaf.
(iii) A difference between the content of RebM and a content of RebE is 1.3% or more per unit mass of a dried leaf.
(iv) a ratio RebM/RebN of the content of RebM to a content of RebN is 1.52 or more per unit mass of a dried leaf.
(v) a ratio RebM/stevioside of the content of RebM to the content of stevioside is 1.83 or more per unit mass of a dried leaf.

The sentence that "the content of RebM is 1.8% or more per unit mass of a dried leaf" in the plant B of the present invention having the chemical feature (i) means, for example, that RebM (for example, 0.9 mg or more) is contained in a proportion of 1.8 mass% or more in a predetermined mass of a dried leaf (for example, 50 mg). The proportion of RebM per unit mass of a dried leaf in this aspect is not limited, and may be, for example, 1.8% or more, 1.9% or more, 1.95% or more, 2.0% or more, 2.1% or more, 2.2% or more, 2.3% or more, 2.4% or more, or 2.5% or more. The upper limit of the proportion of RebM per unit mass of a dried leaf is not particularly limited, and may be, for example, 10%, 8%, 7%, 6%, 5%, 4%, 3%, or 2.5%.

As used herein, the "dried leaf" refers to a leaf obtained by drying a fresh leaf of the stevia plant of the present invention and reducing a water content from 3 to 4 wt.%.

The sentence that "the difference between the content of RebM and the content of stevioside is 0.9% or more per unit mass of a dried leaf" in the plant B of the present invention having the chemical feature (ii) means, for example, that the difference between the amount of RebM and the amount of stevioside contained in a predetermined mass of a dried leaf (for example, 50 mg) is 0.9 mass% or more. The difference between the content of RebM and the content of stevioside per unit mass of a dried leaf in this aspect is not limited, and may be, for example, 0.9% or more, 1.0% or more, 1.1% or more, 1.2% or more, 1.3% or more, 1.4% or more, 1.5% or more, 1.6% or more, 1.7% or more, 1.8% or more, 1.9% or more, or 2.0% or more. The upper limit of the difference between the content of RebM and the content of stevioside per unit mass of a dried leaf is not particularly limited, and may be, for example, 5%, 4%, 3%, 2.5%, or 2%.

The sentence that "the difference between the content of RebM and the content of RebE is 1.3% or more per unit mass of a dried leaf" in the plant B of the present invention having the chemical feature (iii) means, for example, that the difference between the amount of RebM and the amount of RebE contained in a predetermined mass of a dried leaf (for example, 50 mg) is 1.3 mass% or more. The difference between the amount of RebM and the amount of RebE per unit mass of a dried leaf in this aspect is not limited, and may be, for example, 1.3% or more, 1.4% or more, 1.5% or more, 1.6% or more, 1.7% or more, 1.8% or more, 1.9% or more, 2.0% or more, 2.1% or more, 2.2% or more, 2.3% or more, or 2.4% or more. The upper limit of the difference between the content of RebM and the content of RebE per unit mass of a dried leaf is not particularly limited, and may be, for example, 5%, 4%, 3%, 2.5%, or 2%.

The sentence that "the ratio RebM/RebN of the content of RebM to the content of RebN is 1.52 or more per unit mass of a dried leaf" in the plant B of the present invention having the chemical feature (iv) means, for example, that the ratio of RebM to RebN contained in a predetermined mass of a dried leaf (for example, 50 mg) is 1.52 or more. The RebM/RebN per unit mass of a dried leaf in this aspect is not limited, and may be, for example, 1.52 or more, 1.6 or more, 1.7 or more, 1.75 or more, 1.8 or more, 1.83 or more, 1.85 or more, 1.90 or more, 1.95 or more, 2.0 or more, or 2.1 or more. The upper limit of the ratio of RebM to RebN per unit mass of a dried leaf is not particularly limited, and may be, for example, 20.0, 10.0, 9.0, 8.0, 7.0, 6.0, or 5.0.

The sentence that "the ratio RebM/ST of the content of RebM to the content of stevioside is 1.83 or more per unit mass of a dried leaf" in the plant B of the present invention having the chemical feature (v) means, for example, that the ratio of RebM to stevioside contained in a predetermined mass of a dried leaf (for example, 50 mg) is 1.83 or more. The RebM/ST per unit mass of a dried leaf in this aspect is not limited, and may be, for example, 1.83 or more, 1.9 or more, 2.0 or more, 2.1 or more, 2.4 or more, 2.5 or more, 2.8 or more, 3.0 or more, 3.1 or more, 3.3 or more, 3.5 or more, 3.7 or more, 3.9 or more, 4.0 or more, 4.5 or more, 5.0 or more, 5.5 or more, or 6.0 or more. The upper limit of the ratio of RebM to stevioside per unit mass of a dried leaf is not particularly limited, and may be, for example, 20.0, 10.0, 9.0, 8.0, 7.0, 6.0, or 5.0.

In some aspects, the plant B of the present invention may have a plurality of the chemical features described above. In this aspect, the number of chemical features may be any of from 2 to 5, i.e., 2, 3, 4, or 5.

In a specific aspect, the present invention provides a stevia plant having at least one of the genetic features (1) to (5) and at least one of the chemical features (i) to (v) (hereinafter, sometimes referred to as "plant C of the present invention" or "stevia plant C of the present invention").

In the present specification, the plants A to C of the present invention are sometimes collectively referred to as "plant of the present invention".

The steviol glycosides such as RebD, RebE, RebM and RebN can be extracted in the form of an extraction liquid by reacting fresh leaves or dried leaves of the plant of the present invention with an appropriate solvent (an aqueous solvent such as water or an organic solvent such as alcohol, ether and acetone). For conditions for extraction and the like, reference can be made to the method described in Ohta et al., J. Appl. Glycosci., Vol. 57, No. 3, 199 to 209 (2010) or WO2010/038911, or the method described in Examples mentioned below.

Further, individual steviol glycosides such as RebD, RebE, RebM, and RebN can be purified from the thus obtained extraction liquid by using a known method such as a gradient of ethyl acetate or any other organic solvent: water, high performance liquid chromatography (HPLC), gas chromatography, time-of-flight mass spectrometry (TOF-MS), or ultra (high) performance liquid chromatography (UPLC).

The content of the steviol glycoside such as RebD, RebE, RebM or RebN can be measured by the method described in Ohta et al. or WO2010/038911, or by the method described in Examples mentioned below. Specifically, the content of the steviol glycoside can be measured by, for example, sampling a fresh leaf from the stevia plants A to C of the present invention and performing LC/MS-MS.

The plant of the present invention may be a plant obtained by a genetic modification approach or a progeny plant thereof (hereinafter, referred to as the "genetically modified plant"), or may be a plant obtained by a non-genetic modification approach or a progeny plant thereof (hereinafter, referred to as the "non-genetically modified plant"). Examples of the "non-genetic modification approach" include a method of inducing a mutation in a gene of a host cell (or a host plant) without introduction of a foreign gene, in addition to crossing and selfing. Examples of such a method include a method of allowing a mutagen to act on a plant cell. Examples of such a mutagen include ethylmethanesulfonate (EMS) and sodium azide. For example, EMS can be used at a concentration such as 0.1%, 0.2%, 0.3%, 0.4%, 0.5%, 0.6%, 0.7%, 0.8%, 0.9%, or 1.0% to treat a plant cell. The treatment time is from 1 to 48 hours, from 2 to 36 hours, from 3 to 30 hours, from 4 to 28 hours, from 5 to 26 hours, or from 6 to 24 hours. The procedures themselves of the treatment are known in the art and can be performed, e.g., by dipping a water-absorbed seed obtained through a water absorption process in a treatment solution containing the mutagen at the concentration described above for the treatment time described above.

An alternative example of the non-genetic modification approach can be a method of irradiating a plant cell with radiation or light beam such as X ray, γ ray, ion beam (such as heavy ion beam) or ultraviolet ray. In this case, for instance, a cell irradiated using an appropriate dose (ultraviolet lamp intensity, distance, and time) of ultraviolet ray is cultured in a selective medium or the like, and then, a cell, a callus, or a plant having the trait of interest can be selected. In this operation, the irradiation intensity is from 0.01 to 100 Gr, from 0.03 to 75 Gr, from 0.05 to 50 Gr, from 0.07 to 25 Gr, from 0.09 to 20 Gr, from 0.1 to 15 Gr, from 0.1 to 10 Gr, from 0.5 to 10 Gr, or from 1 to 10 Gr. The irradiation distance is from 1 cm to 200 m, from 5 cm to 100 m, from 7 cm to 75 m, from 9 cm to 50 m, from 10 cm to 30 m, from 10 cm to 20 m, or from 10 cm to 10 m. The irradiation time is from 1 minute to 2 years, from 2 minutes to 1 year, from 3 minutes to 0.5 years, from 4 minutes to 1 month, from 5 minutes to 2 weeks, or from 10 minutes to 1 week. The irradiation intensity, distance and time differ depending on the type of radiation or the state of the subject to be irradiated (cell, callus, or plant) and can be appropriately adjusted by those skilled in the art.

Approaches such as cell fusion, another culture (haploid induction), and remote crossing (haploid induction) are also known in the art.

In general, plant cells may involve a mutation during culture. Therefore, it is preferred to regenerate a plant individual, for more stably maintaining the trait.

The scope of the present invention does not exclude a plant obtained by the ex-post facto genetic modification (e.g., genome editing) with the plant of the present invention as a host (e.g., a plant further provided with another trait by genetic modification with the plant of the present invention as a host).

The plant of the present invention includes not only whole plants but also parts and derivatives thereof. Thus, the plants of the present invention may include organs of plants (e.g. leaves, petals, stems, roots, seeds, etc.), tissues of plants (e.g. epidermis, phloem, parenchyma, xylem, vascular bundle, palisade tissue, spongiform tissue, etc.), and various forms of plant cells (e.g. suspension-cultured cells), protoplasts, leaf sections, callus, etc.

The plant of the present invention may also include a tissue culture and a cultured plant cell. This is because a plant can be regenerated by culturing such a tissue culture or a plant cell culture. Examples of a regenerable form of the plant of the present invention include, but are not limited to, embryos, meristematic cells, pollen, leaves, roots, root tips, petals, protoplasts, leaf sections, and callus.

### 2. Method of producing plant of present invention

In another embodiment, the present invention provides a method of producing the stevia plant of the present invention (hereinafter, sometimes referred to as "production method A of the present invention"), the method including a step of crossing the stevia plant of the present invention with a second stevia plant. The stevia plant produced by the method can have the same phenotype and genetic features as the plant of the present invention.

The ranges of the contents of the respective components and the mass ratio, the combinations of the respective features (chemical features and/or genetic features), and the like in the plant obtained by the production method A of the present invention are as described above for the plant of the present invention.

In the production method A of the present invention, the "crossing" means that any one of the plants of the present invention (first generation (S1)) is crossed with a second plant (S1) to obtain a progeny plant thereof (plant produced by the production method of the present invention (second generation (S2)). The crossing method is preferably backcross. The "backcross" is, for example, an approach of further crossing a progeny plant (S2) generated between the plant of the present invention and the second plant, with the plant of the present invention (i.e., a plant having the genetic feature of the present invention) (S1) to produce a plant having the genetic feature of the present invention. When the second plant (S1) for use in the production method of the present invention has the same phenotype and genetic feature as those of the plant of the present invention, the crossing is substantially backcross. The crossing is preferably performed over two or more generations, but, when the genetic feature is heterozygosity or the like, plants having a desired combination of genetic features may be obtained in one generation.

Alternatively, the plant of the present invention can also be produced by selfing. The selfing can be performed by the self-pollination of the stamen pollen of the plant of the present invention with the pistil of the plant of the present invention.

Since the plant produced by the production method of the present invention has the same phenotype and genetic feature as that of the plant of the present invention, the plant produced by the production method of the present invention can be further crossed with a third stevia plant to produce a stevia plant having a phenotype equivalent to that of the plant of the present invention.

In an alternative aspect, the plant of the present invention may be produced by regenerating a plant by the culture of the tissue culture or the cultured plant cell mentioned above. The culture conditions are the same as those for culturing a tissue culture or a cultured plant cell of the wild-type stevia plant and are known in the art (Protocols for In Vitro cultures and secondary metabolite analysis of aromatic and medicinal plants, Method in molecular biology, vol. 1391, pp. 113-123).

In still another aspect, the plant of the present invention can also be produced by introducing the mutation of the present invention into a genome of a stevia plant. Therefore, the present invention provides a method of producing the stevia plant of the present invention (hereinafter sometimes referred to as "production method B of the present invention"), the method including a step of introducing at least one of the following mutations into a genome of a stevia plant.
(1) A mutation of the sequence of a portion corresponding to positions 150 to 151 of SEQ ID NO: 1 to T.
(2) A mutation of the base at a position corresponding to position 151 of SEQ ID NO: 2 to TAGGTGGTGACACTGTAGC.
(3) A mutation of the base at a position corresponding to position 151 of SEQ ID NO: 3 to A.
(4) A mutation of the base at a position corresponding to position 151 of SEQ ID NO: 4 to A.
(5) A mutation of the base at a position corresponding to position 151 of SEQ ID NO: 5 to A.

The mutation may be introduced by a genetic modification approach or a non-genetic modification approach. Examples of the genetic modification approach include a method of introducing a desired mutation into a genome of a host cell (or host plant) by genome editing or the like. The non-genetic modification approach is as described above for the plant of the present invention.

### 3. Method of screening for plant of present invention

The plant of the present invention or the plant having the same phenotype and/or genetic feature as that/those of the plant of the present invention can be screened for by detecting the genetic feature of the present invention from a tissue of the plant. In this context, the "screening" means that the plant of the present invention is discriminated from the other plants to select the plant of the present invention.

Thus, in another aspect, the present invention provides a method of screening for the stevia plant having at least one of the chemical features (i) to (v) (hereinafter sometimes referred to as "screening method of the present invention), the method including a step of detecting, in a test stevia plant, the presence and/or absence of at least one of the genetic features (1) to (5).

The screening method of the present invention may further include a step of selecting a plant in which the presence of at least one of the above-mentioned genetic features has been detected, from among test plants.

The presence of the genetic feature of the invention can be determined, for example, by detecting:
(I) the presence of the allele in which the sequence of the portion corresponding to positions 150 to 151 of SEQ ID NO: 1 is T (e.g., the allele containing the nucleotide sequence of SEQ ID NO: 14, 15, 16 or 17; hereinafter sometimes referred to as "allele i");
(II) the presence of the allele in which the base at the position corresponding to position 151 of SEQ ID NO: 2 is TAGGTGGTGACACTGTAGC (e.g., the allele containing the nucleotide sequence of SEQ ID NO: 18, 19, 20 or 21; hereinafter sometimes referred to as "allele ii");
(III) the presence of the allele in which the base at the position corresponding to position 151 of SEQ ID NO: 3 is A (e.g., the allele containing the nucleotide sequence of SEQ ID NO: 22, 23, 24 or 25; hereinafter sometimes referred to as "allele iii");
(IV) the presence of the allele in which the base at the position corresponding to position 151 of SEQ ID NO: 4 is A (e.g., the allele containing the nucleotide sequence of SEQ ID NO: 26, 27, 28 or 29; hereinafter sometimes referred to as "allele iv"), and/or
(V) the absence of an allele other than the allele in which the base at the position corresponding to position 151 of SEQ ID NO: 5 is A (e.g., the allele containing the nucleotide sequence of SEQ ID NO: 30, 31, 32, or 33; hereinafter sometimes referred to as "allele v").

The absence of the genetic feature of the present invention can be determined, for example, by detecting:
(i) the absence of the allele i;
(ii) the absence of the allele ii;
(iii) the absence of the allele iii;
(iv) the absence of the allele iv; and/or
(v) the presence of an allele other than the allele v.

Specific examples of a method of detecting the genetic feature of the present invention include, but are not limited to, PCR method, TaqMan PCR method, sequencing method, microarray method, invader method, TILLING method, RAD method, RFLP method, PCR-SSCP method, AFLP method, SSLP method, CAPS method, dCAPS method, ASO method, ARMS method, DGGE method, CCM method, DOL method, MALDI-TOF/MS method, TDI method, padlock probe method, molecular beacon method, DASH method, UCAN method, ECA method, PINPOINT method, PROBE method, VSET method, Survivor assay, Sniper assay, Luminex assay, GOOD method, LCx method, SNaPshot method, Mass ARRAY method, pyrosequencing method, SNP-IT method, and melting curve analysis method.

In one aspect, the genetic feature (1) of the present invention can be detected by, for example, the dCAPS method using the following primer set and restriction enzyme.

### • Primer set:

A primer set including: a forward primer containing a sequence of from 15 to 25 continuous bases in length from the 3' end of SEQ ID NO: 34; and a reverse primer containing a sequence (e.g., SEQ ID NO: 35) complementary to any sequence of 15 or more continuous bases located closer to the 3' end side than position 26 of SEQ ID NO: 36 or 37.

### • Restriction enzyme: Hpy188I or an isoschizomer thereof

In one aspect, the genetic feature (4) of the present invention can be detected by, for example, the dCAPS method using the following primer set and restriction enzyme.

### • Primer set:

A primer set including: a forward primer containing a sequence (for example, SEQ ID NO: 40) of any 15 or more continuous located closer to the 5' end than position 78 of SEQ ID NO: 42 or 45; and a reverse primer containing a sequence of from 15 to 25 continuous bases in length from the 3' end of SEQ ID NO: 41.

### • Restriction enzyme: NdeI or an isoschizomer thereof

In one aspect, the genetic feature (5) of the present invention can be detected by, for example, the dCAPS method using the following primer set and restriction enzyme.

### • Primer set:

A primer set including: a forward primer containing a sequence of from 15 to 28 continuous bases in length from the 3' end of SEQ ID NO: 46; and a reverse primer containing a sequence (e.g., SEQ ID NO: 47) complementary to any sequence of 15 or more continuous bases located closer to the 3' end side than position 29 of SEQ ID NO: 48 or 51.

### • Restriction enzyme: MfeI or an isoschizomer thereof

In one aspect, the genetic feature (2) of the present invention can be detected using, for example, the following primer set.

### • Primer set:

A primer set including: a forward primer containing a sequence of from 15 to 25 continuous bases in length from the 3' end of SEQ ID NO: 52; and a reverse primer containing a sequence (e.g., SEQ ID NO: 53) complementary to any sequence of 15 or more continuous bases located closer to the 3' end side than position 26 of SEQ ID NO: 54 or 55.

The sequences of the primers can be optimized within ranges in which they satisfy the above conditions. For optimizing the primer design, see, e.g., Sambrook and Russell, "Molecular Cloning: A Laboratory Manual" 3rd Edition (2001), Cold Spring Harbor Laboratory Press. Each of the primers may have a length of from 15 to 50 bases, from 18 to 48 bases, from 20 to 45 bases, from 30 to 40 bases, or the like. It is also possible to design a primer set other than those described above based on the genetic features of the present invention and to select a restriction enzyme corresponding to the primer set.

In a specific aspect, the genetic feature (1), (4) or (5) of the present invention can be detected by, for example, the dCAPS method using a primer set having the following sequence and a restriction enzyme.

**[Table 1]**

| Table 1: Examples of combinations of primer sets and restriction enzymes for detecting genetic feature (1), (4) or (5) | | | |
|---|---|---|---|
| Genetic feature | Forward primer | Reverse primer | Restriction enzyme |
| (1) | SEQ ID NO: 34 | SEQ ID NO: 35 | Hpy188I |
| (4) | SEQ ID NO: 40 | SEQ ID NO: 41 | NdeI |
| (5) | SEQ ID NO: 46 | SEQ ID NO: 47 | MfeI |

The combinations of the primer sets and the restriction enzymes described above are merely examples, and those skilled in the art can find other combinations of primer sets and restriction enzymes that can detect the genetic features of the present invention.

The screening method of the present invention may further include a step of measuring content(s) of RebM, stevioside, RebE and/or RebN in a tissue (e.g., a leaf) of a test stevia plant in which at least one of the genetic features (1) to (5) of the present invention has been detected. The measurement of the content(s) of RebM, stevioside, RebE and/or RebN is as described in the section of the plant of the present invention. In this aspect, an individual having high RebM content, difference between the RebM content and the stevioside content, difference between the RebM content and the RebE content, ratio of the RebM content to the RebN content, and/or ratio of the RebM content to the stevioside content may be selected from the test stevia plants in which at least one of the genetic features (1) to (5) of the present invention has been detected, and crossed with another stevia plant, and the screening method of the present invention may be applied to the resulting progeny plant. Thus, the screening method of the present invention may include one or more of the following steps:
(i) detecting at least one of the genetic features (1) to (5) of the present invention from the genome of the test stevia plant;
(ii) measuring the content(s) of RebM, stevioside, RebE and/or RebN in a tissue of the test stevia plant in which at least one of the genetic features (1) to (5) of the present invention has been detected;
(iii) selecting an individual having high RebM content, difference between the RebM content and the stevioside content, difference between the RebM content and the RebE content, ratio of the RebM content to the RebN content, and/or ratio of the RebM content to the stevioside content from the stevia plants in which at least one of the genetic features (1) to (5) of the present invention has been detected;
(iv) crossing the selected individual having high RebM content, difference between the RebM content and the stevioside content, difference between the RebM content and the RebE content, ratio of the RebM content to the RebN content, and/or ratio of the RebM content to the stevioside content with another stevia plant;
(v) detecting at least one of the genetic features (1) to (5) of the present invention from a genome of the progeny plant obtained by crossing;
(vi) measuring the content(s) of RebM, stevioside, RebE and/or RebN in a tissue of the progeny plant in which at least one of the genetic features (1) to (5) of the present invention has been detected; and
(vii) selecting an individual having high RebM content, difference between the RebM content and the stevioside content, difference between the RebM content and the RebE content, ratio of the RebM content to the RebN content, and/or ratio of the RebM content to the stevioside content from the progeny plants in which at least one of the genetic features (1) to (5) of the present invention has been detected.

The selected individual having high RebM content, difference between the RebM content and the stevioside content, difference between the RebM content and the RebE content, ratio of the RebM content to the RebN content, and/or ratio of the RebM content to the stevioside content may be, for example, up to top 50%, up to top 40%, up to top 30%, up to top 20%, up to top 10%, up to top 5%, up to top 4%, up to top 3%, up to top 2%, or up to top 1%, in terms of high RebM content, difference between the RebM content and the stevioside content, difference between the RebM content and the RebE content, ratio of the RebM content to the RebN content, and/or ratio of the RebM content to the stevioside content from the test stevia plants in which at least one of the genetic features (1) to (5) of the present invention has been detected. The other stevia plant to be crossed may or may not have the genetic features (1) to (5) of the present invention. The steps (iv) to (vii) can be repeated a plurality of times in the above aspect. This enables screening for a stevia plant having a higher individual having high RebM content, difference between the RebM content and the stevioside content, difference between the RebM content and the RebE content, ratio of the RebM content to the RebN content, and/or ratio of the RebM content to the stevioside content.

In the screening method of the present invention, the test stevia plant may be a natural plant of a non-genetically modified plant. The non-genetically modified plant is as described in the section of the plant of the present invention.

In the screening method of the present invention, the test stevia plant may include a stevia plant subjected to a mutagenesis treatment and a progeny plant thereof. The mutagenesis is as described in the section of the plant of the present invention, and includes a treatment with a mutagen, a treatment with radiation or light irradiation, and the like.

The present invention also provides the primer sets described above and combinations thereof, for example, the primer sets shown in Table 1 above and combinations of these primer sets. The present invention further provides primer sets capable of amplifying a region having a nucleotide sequence selected from SEQ ID NOs: 14 to 33 and 56 to 70 by PCR, for example, a primer set including a forward primer containing the nucleotide sequence of SEQ ID NO: 6 and a reverse primer containing the nucleotide sequence of SEQ ID NO: 7, a primer set including a forward primer containing the nucleotide sequence of SEQ ID NO: 8 and a reverse primer containing the nucleotide sequence of SEQ ID NO: 9, a primer set including a forward primer containing the nucleotide sequence of SEQ ID NO: 10 and a reverse primer containing the nucleotide sequence of SEQ ID NO: 11, and a primer set including a forward primer containing the nucleotide sequence of SEQ ID NO: 12 and a reverse primer containing the nucleotide sequence of SEQ ID NO: 13.

Further, the present invention provides a probe capable of detecting the presence and/or absence of the genetic feature of the present invention (hereinafter sometimes referred to as "probe of the present invention"). The probe of the present invention may have a structure suitable for various methods of detecting the presence and/or absence of the genetic feature of the present invention (e.g., a real-time PCR method such as a TaqMan PCR method). For example, the probe of the present invention may contain a nucleotide sequence complementary to a portion of the genome containing the mutation site of the present invention. Non-limiting examples of such a probe include those containing a sequence complementary to a nucleotide sequence selected from SEQ ID NOs: 14 to 16, 18 to 20, 22 to 24, 26 to 28, 30 to 32, and 56 to 70. Among these sequences, SEQ ID NOs: 14 to 16, 18 to 20, 22 to 24, 26 to 28, and 30 to 32 are specific to an allele containing the mutation of the present invention, and SEQ ID NOs: 56 to 70 are specific to an allele not containing the mutation of the present invention. The presence of the genetic feature of the present invention can be detected through detection of both an allele containing the mutation of the present invention and an allele not containing the mutation of the present invention, and the absence of the genetic feature of the present invention can be detected through detection of only an allele containing the mutation of the present invention or detection of only an allele not containing the mutation of the present invention. The probe of the present invention preferably has a label. Non-limiting examples of such a label include fluorescent labels, luminescent labels, radioactive labels, dyes, enzymes, quenchers, and binding moieties with detectable labels. In a specific aspect, the probe of the present invention has a polynucleotide containing a nucleotide sequence complementary to the nucleotide sequence selected from SEQ ID NOs: 14 to 16, 18 to 20, 22 to 24, 26 to 28, 30 to 32, and 56 to 70, and a label.

The present invention further provides a kit including the primer set described above and a restriction enzyme corresponding to the primer set. In a specific aspect, the kit of the present invention includes the primer set as shown in Table 1 above and the restriction enzyme corresponding to the primer set.

The present invention also provides a kit including a primer set capable of amplifying a region having a nucleotide sequence selected from the group consisting of SEQ ID NOs: 14 to 33 and 56 to 70 by PCR, and the probe of the present invention corresponding to the primer set.

The primer set, probe, and kit can be used for detecting the genetic features (1) to (5) of the present invention, used in the screening method of the present invention, and the like. In addition, the primer set and kit may include an instruction including explanations for the detection of the genetic features (1) to (5) of the present invention and the screening method of the present invention, for example, an instruction manual, site information including information on usage (for example, URL and a two dimensional code), a medium recording the information on usage (for example, a flexible disk, a CD, a DVD, a Blu-ray disk, a memory card, or a USB memory), and the like.

In some aspects, the present invention provides a screening kit for the stevia plant of the present invention, including a reagent for detecting the presence and/or absence of at least one of the genetic features (1) to (5). The reagent may include a primer and/or a probe for use in a CAPS method, a dCAPS method or a TaqMan PCR method. In a specific aspect, the reagent for detecting the presence and/or absence of at least one of the genetic features (1) to (5) includes a combination of a primer set for detecting at least one of the genetic features (1) to (5) by the dCAPS method and a restriction enzyme, for example, the combination of the primer set and the restriction enzyme as shown in Table 1, or a combination of a primer set amplifying the mutation site of the present invention (for example, a site including a sequence selected from SEQ ID NOs: 14 to 33) and a probe having a nucleotide sequence complementary to a site related to at least one of the genetic features (1) to (5) (for example, a site including a sequence selected from SEQ ID NOs: 14 to 16, 18 to 20, 22 to 24, and 26 to 28, 30 to 32) that can be used in a TaqMan PCR method or the like).

### 4. Dead tissue or dead cell of present invention

In a further aspect, the present invention provides a dead tissue or cell of the stevia plant of the present invention, a stevia plant selected by the screening method of the present invention, or a stevia plant produced by the production method of the present invention (hereinafter, also referred to as "dead tissue or dead cell of the present invention"). The term "dead" means a state in which there is no ability to reproduce, proliferate, reproduce, or grow, and includes a case in which death occurs naturally without artificial manipulation, and a case in which death occurs due to artificial manipulation such as cutting, crushing, heating (including heating via a gas such as air, heating via a liquid such as water, and heating via a vapor such as water vapor), freezing, drying, and freeze-drying. The "dead tissue" means a tissue in which all cells contained therein are dead, and includes a dead plant (a plant in which all cells contained therein are dead). Specific examples of the dead tissue or dead cell of the present invention include dead tissues or cells other than seeds. More specific examples of the dead tissue or dead cell include dead embryos, meristematic cells, pollen, leaves, roots, root tips, petals, protoplasts, leaf sections and callus. A dried leaf is suitably employed. The dead tissue or dead cell of the present invention can be used as a raw material for an extract, a steviol glycoside, a medicament, a flavor, a food or beverage, or the like, which will be described later.

### 5. Method of producing plant-derived extract and product using the extract

In a further aspect, the present invention provides a method of producing a steviol glycoside-containing extract (hereinafter, sometimes referred to as "method of producing an extract of the present invention"), the method including a step of producing an extract from the plant of the present invention, a stevia plant selected by the screening method of the present invention or a stevia plant produced by the production method of the present invention, a dead tissue or dead cell of the plant, or a seed, a leaf (for example, a dried leaf or a fresh leaf), a tissue, a tissue culture or a cell of the plant.

Further, the present invention provides a steviol glycoside-containing extract from the plant of the present invention, a stevia plant selected by the screening method of the present invention or a stevia plant produced by the production method of the present invention, a seed, a leaf (for example, a dried leaf or a fresh leaf), a tissue, a tissue culture or a cell of the plant, or the dead tissue or dead cell of the present invention (hereinafter, sometimes referred to as "extract of the present invention"). The extract of the present invention is preferably produced by the method of producing an extract of the present invention. Furthermore, the present invention provides a method of producing a steviol glycoside (hereinafter, sometimes referred to as "method of producing a steviol glycoside of the present invention"), the method including a step of purifying a steviol glycoside from the extract of the present invention. Specifically, the method of producing a steviol glycoside of the present invention may further include a step of producing a steviol glycoside-containing extract from the stevia plant of the present invention, a stevia plant selected by the screening method of the present invention or a stevia plant produced by the production method of the present invention, or the dead tissue or dead cell of the present invention.

The steviol glycoside-containing extract can be obtained by, for example, reacting a fresh leaf or dried leaf of the plant of the present invention with an appropriate solvent (an aqueous solvent such as water or an organic solvent such as alcohol, ether and acetone). For conditions for extraction and the like, reference can be made to the method described in Ohta et al. or WO2010/038911, or the method described in Examples mentioned below.

The steviol glycoside can be purified from the steviol glycoside-containing extract by using a known method such as a gradient of ethyl acetate or any other organic solvent: water, chromatography (such as high performance liquid chromatography (HPLC), ultra (high) performance liquid chromatography (UPLC) and gas chromatography), or time-of-flight mass spectrometry (TOF-MS).

The type of steviol glycoside is not particularly limited, and includes RebA, RebB, RebC, RebD, RebE, RebF, RebG, RebI, RebJ, RebK, RebM, RebN, RebO, RebQ, RebR, dulcoside A, rubusoside, steviolmonoside, steviolbioside and stevioside. In a preferred aspect, the steviol glycoside contains RebD, RebE, RebM, RebN, stevioside, or a combination thereof.

In some aspects, the extract of the invention has higher RebM content, difference between the RebM content and the stevioside content, difference between the RebM content and the RebE content, ratio of the RebM content to the RebN content, and/or ratio of the RebM content to the stevioside content than that/those in a stevia species that does not have any of the genetic features (2'), (4') and (5) of the present invention.

The extract of the present invention may have about 50% or more, about 100% or more, about 150% or more, about 200% or more, about 250% or more, about 300% or more, about 350% or more, about 400% or more, about 450% or more, about 500% or more, about 550% or more, about 600% or more, about 650% or more, about 700% or more, about 750% or more, about 800% or more, about 850% or more, about 900% or more, about 950% or more, about 1000% or more, about 1050% or more, about 1100% or more, about 1150% or more, about 1200% or more, about 1250% or more, about 1300% or more, about 1350% or more, about 1400% or more, about 1450% or more, about 1500% or more, about 1550% or more, about 1600% or more, about 1650% or more, about 1700% or more, about 1750% or more, about 1800% or more, about 1850% or more, about 1900% or more, about 1950% or more, about 2000% or more, about 2050% or more, or about 2100% or more higher RebM content, difference between the RebM content and the stevioside content, difference between the RebM content and the RebE content, ratio of the RebM content to the RebN content, and/or ratio of the RebM content to the stevioside content than that/those in an extract obtained from a stevia plant that does not have any of the genetic features (2'), (4'), and (5) of the present invention. Here, the extract of the present invention and the extract obtained from the stevia plant that does not have any of the genetic features (2'), (4'), and (5) of the present invention may be obtained by the same method.

By mixing the thus obtained extract of the present invention and/or the steviol glycoside obtained by the method of producing a steviol glycoside of the present invention with another component, a novel food or beverage, sweetener composition, flavor or medicament having an increased steviol glycoside content can be produced. Thus, in another embodiment, the present invention provides a method of producing a food or beverage, a sweetener composition, a flavor, or a medicament, the method including a step of mixing the extract of the present invention and/or the steviol glycoside obtained by the method of producing a steviol glycoside of the present invention with another component such as a raw material for a food or beverage, a sweetener, a flavor, or a medicament. Furthermore, the present invention provides a novel food or beverage, sweetener composition, flavor or medicament with an increased steviol glycoside content, which is obtained by the production method. The "food or beverage" as used herein includes a beverage and a food. Thus, in an embodiment, the present invention provides a novel beverage, food, sweetener composition, flavor or medicament, and also provides a method of producing the beverage, food, sweetener composition, flavor or medicament.

Further, the present invention provides a method of producing a steviol glycoside-containing composition, the method including a step of mixing the extract of the present invention and/or the steviol glycoside obtained by the method of producing a steviol glycoside of the present invention with other component(s). Furthermore, the present invention provides a steviol glycoside-containing composition containing a steviol glycoside and other component(s), obtained by the production method. The "other component(s)" may comprise a component not present in naturally occurring stevia plant or a non-natural component. Thus, the composition may be a non-natural composition. The composition may be, for instance, a pharmaceutical composition, a flavor composition, a beverage composition, or a food composition.

### 6. Nucleotide sequence of plant of present invention

In another aspect, the present invention provides a nucleotide sequence of the plant of the present invention.

The nucleotide sequence of a stevia plant having the genetic feature (1) contains or consists of a nucleotide sequence selected from SEQ ID NOs: 14 to 17. The nucleotide sequence of a stevia plant having the genetic feature (2) contains or consists of a nucleotide sequence selected from SEQ ID NOs: 18 to 21. The nucleotide sequence of a stevia plant having the genetic feature (3) contains or consists of a nucleotide sequence selected from SEQ ID NOs: 22 to 25. The nucleotide sequence of a stevia plant having the genetic feature (4) contains or consists of a nucleotide sequence selected from SEQ ID NOs: 26 to 29. The nucleotide sequence of a stevia plant having the genetic feature (5) contains or consists of a nucleotide sequence selected from SEQ ID NOs: 30 to 33.

### Examples

Hereinafter, examples related to the present invention will be described, but the present invention is not limited to these specific aspects.

### [Example 1] Identification of mutation related to high RebM phenotype

The steviol glycoside contents of stevia lines grown at Suntory World Research Center were measured and genome sequencing was performed. To be specific, 0.25 g of fresh leaves were sampled from an individual of each line and dried by freeze-drying, and 0.05 g of a crushed dry matter was added to a 100-fold amount (5 mL) of pure water. Extraction was performed by an ultrasonic treatment for 20 minutes. The extract was centrifuged and filtered, and then diluted 60 times with 32% acetonitrile to obtain a sample solution. This sample solution (1 mL) was subjected to LC-MS/MS analysis in the MRM mode of LCMS8050 to quantify the concentrations of RebA, RebB, RebC, RebD, RebE, RebF, RebI, RebJ, RebM, RebN and ST. Further, genomic DNA was extracted from the sampled fresh leaves and sequenced by a sequencer (HiSeq 2500, Illumina).

Analysis of the data on the steviol glycoside contents revealed the presence of a line group with a high RebM content (high RebM group). Tables 2 and 3 show steviol glycoside contents of representative lines of a high RebM group, a low RebM group with a relatively low RebM content, and a medium RebM group with a lower RebM content than that in the high RebM group and a higher RebM content than that in the low RebM group. In addition, Table 4 illustrates differences and ratios between the contents of various steviol glycosides.

In the table below, TSG indicates the total steviol glycoside. In addition, the numerical value of each steviol glycoside is wt.% in dried leaves, and the numerical value of TSG is a total value of the numerical values of all the steviol glycosides measured (i.e., RebA, RebB, RebC, RebD, RebE, RebF, RebI, RebJ, RebM, RebN, and ST).

**[Table 2]**

| Table 2: Steviol glycoside contents of each line (1) | | | | | | | |
|---|---|---|---|---|---|---|---|
| | Line name | RebA | RebB | RebC | RebD | RebE | RebF |
| High RebM | A | 6.1061 | 0.0713 | 0.3878 | 4.9443 | 0.1588 | 0.2043 |
| | B | 5.9318 | 0.0728 | 0.3567 | 5.9816 | 0.3316 | 0.2182 |
| | C | 5.7603 | 0.0857 | 0.4346 | 4.7024 | 0.1829 | 0.2030 |
| | D | 3.7856 | 0.0532 | 0.3563 | 5.2035 | 0.3148 | 0.1578 |
| | E | 4.6714 | 0.0633 | 0.4130 | 4.2749 | 0.2026 | 0.1805 |
| | F | 11.414 8 | 0.1386 | 0.8838 | 4.6728 | 0.1706 | 0.4029 |
| | G | 4.2825 | 0.0763 | 0.6470 | 6.4834 | 0.5351 | 0.1877 |
| | H | 4.0637 | 0.0503 | 0.3417 | 5.6136 | 0.4054 | 0.1621 |
| Mediu m RebM | I | 5.8054 | 0.0720 | 0.4037 | 6.2679 | 0.6520 | 0.2313 |
| | J | 4.6333 | 0.0775 | 0.4542 | 5.2286 | 0.3841 | 0.2206 |
| | K | 7.8480 | 0.1044 | 0.6348 | 4.2707 | 0.1798 | 0.2645 |
| | L | 5.9004 | 0.0720 | 0.6896 | 3.9158 | 0.2288 | 0.2185 |
| Low RebM | M | 6.4646 | 0.1084 | 0.5878 | 6.3744 | 0.5104 | 0.2404 |
| | N | 5.8522 | 0.1743 | 0.6302 | 3.6932 | 0.1885 | 0.2024 |
| | O | 5.8362 | 0.0997 | 0.7947 | 5.3401 | 0.5106 | 0.2787 |
| | P | 5.8203 | 0.1324 | 0.8110 | 4.6496 | 0.3615 | 0.2627 |
| | Q | 3.8519 | 0.0863 | 0.4513 | 4.0164 | 0.2646 | 0.1516 |
| | R | 3.1090 | 0.0544 | 0.3416 | 3.5032 | 0.2260 | 0.1164 |
| | S | 5.8705 | 0.1052 | 0.4913 | 3.0450 | 0.1681 | 0.2214 |
| | T | 2.2111 | 0.0611 | 0.4461 | 6.0842 | 1.5745 | 0.1317 |
| | U | 3.7490 | 0.0616 | 0.4791 | 6.2889 | 1.7276 | 0.1716 |
| | V | 1.8513 | 0.0466 | 0.5435 | 6.7681 | 2.4577 | 0.1121 |
| | W | 1.6358 | 0.0462 | 0.5921 | 6.7162 | 2.7529 | 0.1130 |
| | X | 1.4289 | 0.0201 | 0.6238 | 4.5409 | 3.3033 | 0.1054 |

**[Table 3]**

| Table 3: Steviol glycoside contents of each line (2) | | | | | | | |
|---|---|---|---|---|---|---|---|
| | Line name | RebI | RebJ | RebM | RebN | ST | TSG |
| High RebM | A | 0.0420 | 0.0958 | 2.5110 | 1.1622 | 0.4890 | 17.674 4 |
| | B | 0.0510 | 0.1610 | 2.2878 | 1.2029 | 0.3753 | 17.368 2 |
| | C | 0.0387 | 0.0787 | 2.2008 | 1.2325 | 0.5950 | 16.970 6 |
| | D | 0.0260 | 0.0764 | 1.9578 | 1.1161 | 0.6365 | 18.430 3 |
| | E | 0.0291 | 0.0684 | 1.9556 | 1.0664 | 0.6965 | 19.479 7 |
| | F | 0.0916 | 0.2238 | 1.8118 | 1.0366 | 0.5876 | 21.434 9 |
| | G | 0.0360 | 0.2448 | 1.7978 | 4.3150 | 0.8740 | 10.459 6 |
| | H | 0.0464 | 0.1410 | 1.7915 | 1.5870 | 0.4585 | 15.514 6 |
| Mediu m RebM | I | 0.0443 | 0.1839 | 1.6955 | 1.1189 | 0.8931 | 18.239 9 |
| | J | 0.0361 | 0.1156 | 1.6742 | 1.5205 | 0.9164 | 15.980 0 |
| | K | 0.0516 | 0.1006 | 1.6712 | 2.4253 | 0.6890 | 15.261 1 |
| | L | 0.0539 | 0.2778 | 1.6458 | 1.5043 | 0.4590 | 16.172 8 |
| Low RebM | M | 0.0184 | 0.3097 | 1.4969 | 3.0197 | 1.2537 | 12.889 8 |
| | N | 0.0552 | 0.0948 | 1.3184 | 1.3980 | 1.0772 | 16.346 7 |
| | O | 0.0220 | 0.1221 | 1.2393 | 2.8826 | 1.8512 | 13.683 8 |
| | P | 0.0369 | 0.1792 | 1.1610 | 1.8728 | 1.0594 | 15.389 6 |
| | Q | 0.0167 | 0.2621 | 1.1486 | 1.0776 | 0.7028 | 18.977 2 |
| | R | 0.0191 | 0.0438 | 1.0909 | 1.1507 | 0.8045 | 18.197 9 |
| | S | 0.0361 | 0.0902 | 1.0525 | 1.0602 | 0.7493 | 20.384 4 |
| | T | 0.0095 | 0.0917 | 0.6601 | 2.5930 | 2.1171 | 14.661 3 |
| | U | 0.0077 | 0.2163 | 0.5972 | 2.5315 | 2.5999 | 14.684 5 |
| | V | 0.0061 | 0.0718 | 0.5889 | 2.9468 | 2.2816 | 13.621 7 |
| | W | 0.0046 | 0.1021 | 0.4906 | 3.3647 | 2.3795 | 12.029 9 |
| | X | 0.0035 | 0.1257 | 0.2656 | 2.4893 | 2.4831 | 14.966 0 |

**[Table 4]**

| Table 4: Steviol glycoside contents of each line (3) | | | | | |
|---|---|---|---|---|---|
| | Line name | RebM - ST | RebM - RebE | RebM/Reb N | RebM/ST |
| High RebM | A | 2.0220 | 2.3522 | 2.1606 | 5.1350 |
| | B | 1.9124 | 1.9562 | 1.9019 | 6.0954 |
| | C | 1.6059 | 2.0179 | 1.7857 | 3.6992 |
| | D | 1.3213 | 1.6430 | 1.7542 | 3.0759 |
| | E | 1.2591 | 1.7531 | 1.8339 | 2.8078 |
| | F | 1.2242 | 1.6412 | 1.7478 | 3.0833 |
| | G | 0.9239 | 1.2628 | 0.4167 | 2.0571 |
| | H | 1.3330 | 1.3861 | 1.1288 | 3.9071 |
| Mediu m RebM | I | 0.8024 | 1.0435 | 1.5153 | 1.8984 |
| | J | 0.7578 | 1.2900 | 1.1011 | 1.8270 |
| | K | 0.9822 | 1.4914 | 0.6891 | 2.4255 |
| | L | 1.1868 | 1.4171 | 1.0941 | 3.5853 |
| | M | 0.2432 | 0.9865 | 0.4957 | 1.1940 |
| | N | 0.2412 | 1.1299 | 0.9430 | 1.2239 |
| | O | -0.6119 | 0.7287 | 0.4299 | 0.6695 |
| | P | 0.1016 | 0.7996 | 0.6199 | 1.0959 |
| | Q | 0.4458 | 0.8840 | 1.0659 | 1.6342 |
| Low RebM | R | 0.2865 | 0.8649 | 0.9480 | 1.3561 |
| | S | 0.3033 | 0.8844 | 0.9928 | 1.4047 |
| | T | -1.4570 | -0.9144 | 0.2546 | 0.3118 |
| | U | -2.0027 | -1.1303 | 0.2359 | 0.2297 |
| | V | -1.6928 | -1.8688 | 0.1998 | 0.2581 |
| | W | -1.8889 | -2.2623 | 0.1458 | 0.2062 |
| | X | -2.2175 | -3.0376 | 0.1067 | 0.1070 |

The high RebM group, the medium RebM group and the low RebM group were evaluated for the presence of a statistically significant difference in content of each steviol glycoside by Welch's t-test (two sided).

The high RebM group had significantly higher contents of RebI and RebM, significantly higher ratio of the RebM content to the RebN content (RebM/RebN) and ratio of the RebM content to the ST content (RebM/ST), significantly larger RebM - ST (difference between the RebM content and the ST content, the same applies hereinafter) and RebM - RebE, and significantly lower contents of RebE and ST, than those in the medium RebM group and the low RebM group (Table 5).

In terms of the content of RebM, RebM - ST, and RebM - RebE, there were significant differences between the high RebM group and the medium RebM group even when the Welch's t-test (two sided) was performed (no table is indicated).

In the table, the numerical values indicate average values, the symbols "+" and "++" indicate that the high RebM group has a higher content than those in the medium RebM group and the low RebM group at P < 0.05 and P < 0.01, respectively; the symbols "-" and "--" indicate that the high RebM group has a lower content than those in the medium RebM group and the low RebM group at P < 0.05 and P < 0.01, respectively; and the symbol "±" indicates that no significant difference was observed between the contents in both groups at a significance level of 0.05.

**[Table 5]**

| Table 5: Average values of steviol glycoside contents in each group and significant difference | | | |
|---|---|---|---|
| Steviol glycoside | High RebN group | Middle RebN group + Low RebN group | Significant difference |
| RebA | 5.7520 | 4.4918 | ± |
| RebB | 0.0764 | 0.0826 | ± |
| RebC | 0.4776 | 0.5609 | ± |
| RebD | 5.2346 | 5.0440 | ± |
| RebE | 0.2877 | 0.9682 | - |
| RebF | 0.2146 | 0.1901 | ± |
| RebI | 0.0451 | 0.1492 | ++ |
| RebJ | 0.1362 | 0.1492 | ± |
| RebM | 2.0393 | 1.1123 | ++ |
| RebN | 1.5898 | 2.0597 | ± |
| ST | 0.5890 | 1.3948 | - |
| TSG | 17.1665 | 15.7179 | ± |
| RebM - ST | 1.4502 | -0.2825 | ++ |
| RebM - RebE | 1.7515 | 0.1442 | ++ |
| RebM/RebN | 1.5912 | 0.6774 | ++ |
| RebM/ST | 3.7326 | 1.2142 | ++ |

Analysis of a genomic sequence difference between the high RebM lines and the medium RebM lines and low RebM lines revealed a tendency that the high RebM lines have the following genetic features.
(1') Being heterozygous for the allele (SEQ ID NO: 17) in which the sequence of a portion corresponding to positions 150 to 151 of SEQ ID NO: 1 is T.
(2') Being heterozygous for the allele (SEQ ID NO: 21) in which the base at a position corresponding to position 151 of SEQ ID NO: 2 is TAGGTGGTGACACTGTAGC.
(3') Being heterozygous for the allele (SEQ ID NO: 25) in which the base at a position corresponding to position 151 of SEQ ID NO: 3 is A.
(4') Being heterozygous for the allele (SEQ ID NO: 29) in which the base at a position corresponding to position 151 of SEQ ID NO: 4 is A.
(5) Being homozygous for the allele (SEQ ID NO: 33) in which the base at a position corresponding to position 151 of SEQ ID NO: 5 is A.

### [Example 2] Development of high RebM individual detection marker

dCAPS primers for detecting the genetic features (1'), (2'), (4'), and (5) were prepared for efficiently detecting the genetic features specific to the high RebM lines, and it was examined whether the presence or absence of these genetic features can be detected by the dCAPS method for each individual.

The following dCAPS primers and restriction enzymes were used.

**[Table 6]**

| Table 6: Sequences of dCAPS primers and restriction enzymes | | | |
|---|---|---|---|
| Geneti c featur e | Forward primer | Reverse primer | Restrictio n enzyme |
| (1') | TCTAAGTCTGGTCTAGTA CATCTTA(SEQ ID NO: 34) | GTCCTAGCTACTTTAGAT TTATTTA(SEQ ID NO: 35) | Hpy188I |
| (2') | CATTCTAGCCATCATCGC CATCACG(SEQ ID NO: 52) | TCAGTCGCACTCCAATCA TCTAAACC(SEQ ID NO: 53) | - |
| (4') | GATTAATCGGGAGTAATC GGAACTAGG(SEQ ID NO: 40) | AGGTTTTCTTAGGAACCC TCCCATA(SEQ ID NO: 41) | NdeI |
| (5) | GTATGGTTGTTATCAAGA ACTTCTACTG(SEQ ID NO: 46) | TTACGTTATGTATATAAA AATACGTTCAA(SEQ ID NO: 47) | MfeI |

Each genetic feature was detected by the dCAPS method as follows. First, genomic DNA was extracted from a fresh leaf of each individual tested in Example 1, and PCR was performed using the above dCAPS primers corresponding to each genetic feature.

The restriction enzyme corresponding to each of the genetic features (4') and (5) was added to the PCR product, and enzyme reaction was performed at 37°C. The restriction enzyme-treated product of the PCR product was subjected to electrophoresis using a microchip-type electrophoresis device LabChip GX Touch HT (PerkinElmer), and the presence or absence of the genetic feature was determined based on the obtained band pattern.

With respect to the genetic features (4') and (5), an individual in which only a band of a degraded product was observed was determined as ○, an individual in which only a band of a non-degraded product was observed was determined as ×, and an individual in which both bands of a degraded product and a non-degraded product were observed was determined as △. With respect to the genetic feature (2'), an individual in which only a PCR product of about 206 bp was observed was determined as ○, an individual in which only a PCR product of about 188 bp was observed was determined as ×, and an individual in which both a PCR product of about 206 bp and a PCR product of about 188 bp were observed was determined as △. The results are shown in the table below.

**[Table 7]**

| Table 7: Retention status of genetic features in each line | | | | |
|---|---|---|---|---|
| Type | Line name | Genetic feature | | |
| | | (2') | (4') | (5) |
| | A | △ | △ | △ |
| | B | △ | △ | △ |
| High RebM | C | △ | △ | ○ |
| | D | △ | △ | ○ |
| | E | △ | △ | × |
| | F | △ | △ | ○ |
| | G | × | △ | △ |
| | H | △ | △ | ○ |
| Medium RebM | I | △ | × | △ |
| | J | × | × | △ |
| | K | × | × | △ |
| | L | △ | × | △ |
| | M | × | × | × |
| | N | × | × | △ |
| | O | × | △ | △ |
| | P | △ | △ | △ |
| | Q | △ | × | △ |
| Low RebM | R | × | × | × |
| | S | × | × | △ |
| | T | × | × | × |
| | U | × | × | △ |
| | V | × | × | × |
| | W | × | × | × |
| | X | × | × | △ |

The genetic feature (2') was heterozygous, and thus an individual in which both a PCR product at about 206 bp and a PCR product at about 188 bp were observed was determined as having the genetic feature. The genetic feature (4') was heterozygous, and thus an individual in which both bands of a degraded product and a non-degraded product were observed was determined as having the genetic feature. The genetic feature (5) was homozygous, and thus an individual in which a band of a degraded product was observed was determined as having the genetic feature. As a result, the genetic features (2'), (4') and (5) tended to be detected in the high RebM lines. On the other hand, in the medium RebM lines and the low RebM lines, the genetic features (2'), (4'), and (5) tended not to be detected.

[Sequence Listing]

## Claims

1. A stevia plant having at least one of the following genetic features (1) to (5).
(1) Being homozygous or heterozygous for the allele in which the sequence of a portion corresponding to positions 150 to 151 of SEQ ID NO: 1 is T.
(2) Being homozygous or heterozygous for the allele in which the base at a position corresponding to position 151 of SEQ ID NO: 2 is TAGGTGGTGACACTGTAGC.
(3) Being homozygous or heterozygous for the allele in which the base at a position corresponding to position 151 of SEQ ID NO: 3 is A.
(4) Being homozygous or heterozygous for the allele in which the base at a position corresponding to position 151 of SEQ ID NO: 4 is A.
(5) Being homozygous for the allele in which the base at a position corresponding to position 151 of SEQ ID NO: 5 is A.

2. A stevia plant having at least one of the following chemical features (i) to (v).
(i) A content of rebaudioside (Reb) M is 1.8% or more per unit mass of a dried leaf.
(ii) A difference between the content of RebM and a content of stevioside is 0.9% or more per unit mass of a dried leaf.
(iii) A difference between the content of RebM and a content of RebE is 1.3% or more per unit mass of a dried leaf.
(iv) a ratio RebM/RebN of the content of RebM to a content of RebN is 1.52 or more per unit mass of a dried leaf.
(v) a ratio RebM/stevioside of the content of RebM to the content of stevioside is 1.83 or more per unit mass of a dried leaf.

3. The plant according to claim 1 or 2, wherein the plant comprises a stevia plant subjected to a mutagenesis treatment and a progeny plant thereof.

4. A dead tissue or dead cell of the plant according to any one of claims 1 to 3.

5. A seed, tissue, dried leaf, tissue culture, or cell of the plant according to any one of claims 1 to 3.

6. The tissue, tissue culture, or cell according to claim 5, which is selected from embryos, meristematic cells, pollen, leaves, roots, root tips, petals, protoplasts, leaf sections and callus.

7. A method of producing the stevia plant according to any one of claims 1 to 3, the method comprising a step of crossing the plant according to any one of claims 1 to 3 with a second stevia plant.

8. A method of producing the stevia plant according to any one of claims 1 to 3, the method comprising a step of introducing, into a genome of a stevia plant, at least one of the following mutations.
(1) A mutation of the sequence of a portion corresponding to positions 150 to 151 of SEQ ID NO: 1 to T.
(2) A mutation of the base at a position corresponding to position 151 of SEQ ID NO: 2 to TAGGTGGTGACACTGTAGC.
(3) A mutation of the base at a position corresponding to position 151 of SEQ ID NO: 3 to A.
(4) A mutation of the base at a position corresponding to position 151 of SEQ ID NO: 4 to A.
(5) A mutation of the base at a position corresponding to position 151 of SEQ ID NO: 5 to A.

9. An extract of the plant according to any one of claims 1 to 3, of the dead tissue or dead cell according to claim 4, of the seed, tissue, dried leaf, tissue culture, or cell according to claim 5, or of the tissue, tissue culture, or cell according to claim 6.

10. A method of producing a steviol glycoside-containing extract, the method comprising a step of obtaining an extract from the plant according to any one of claims 1 to 3, from the dead tissue or dead cell according to claim 4, from the seed, tissue, dried leaf, tissue culture, or cell according to claim 5, or from the tissue, tissue culture, or cell according to claim 6.

11. A method of producing a steviol glycoside, the method comprising a step of purifying the steviol glycoside from the extract according to claim 9.

12. A method of producing a food or beverage, a sweetener composition, a flavor, or a medicament, the method comprising:
a step of providing an extract of the plant according to any one of claims 1 to 3, an extract of the dead tissue or dead cell according to claim 4, an extract of the seed, tissue, dried leaf, tissue culture, or cell according to claim 5, an extract of the tissue, tissue culture, or cell according to claim 6, or the extract according to claim 9; and
a step of adding the extract to a raw material for the food or beverage, the sweetener composition, the flavor, or the medicament.

13. A food or beverage, a sweetener composition, a flavor, or a medicament, comprising an extract of the plant according to any one of claims 1 to 3, an extract of the dead tissue or dead cell according to claim 4, an extract of the seed, tissue, dried leaf, tissue culture, or cell according to claim 5, an extract of the tissue, tissue culture, or cell according to claim 6, or the extract according to claim 9.

14. A method of producing a food or beverage, a sweetener composition, a flavor, or a medicament, the method comprising:
a step of purifying a steviol glycoside from the extract according to claim 9; and
a step of adding the purified steviol glycoside to a raw material for the food or beverage, the sweetener composition, the flavor, or the medicament.

15. A method of screening for a stevia plant having at least one of the chemical features (i) to (v) recited in claim 2, the method comprising a step of detecting, in a test stevia plant, the presence and/or absence of at least one of the genetic features (1) to (5) recited in claim 1.

16. A screening kit for a stevia plant having at least one of the chemical features (i) to (v) recited in claim 2, the kit comprising a reagent for detecting the presence and/or absence of at least one of the genetic features (1) to (5) recited in claim 1.
